Europäisches Patentamt
European Patent Office
Office européen des brevets

(19) (11) Veröffentlichungsnummer: **0 164 704**
**B1**

(12) # EUROPÄISCHE PATENTSCHRIFT

(45) Veröffentlichungstag der Patentschrift: **06.12.89**

(51) Int. Cl.⁴: **C 07 D 475/14**

(21) Anmeldenummer: **85107062.3**

(22) Anmeldetag: **07.06.85**

ERRATUM

(SEITE, SPALTE, ZEILE)
(PAGE, COLUMN, LINE)
(PAGE, COLONNE, LIGNE)

| DIE TEXTSTELLE:<br>TEXT PUBLISHED:<br>LE PASSAGE SUIVANT: | | | LAUTET BERICHTIGT:<br>SHOULD READ:<br>DEVRAIT ETRE LU: |
|---|---|---|---|
| 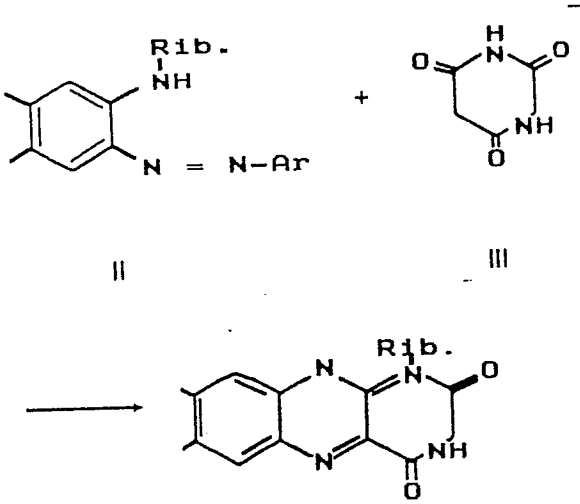<br>II III I | 2 | 10/19 | 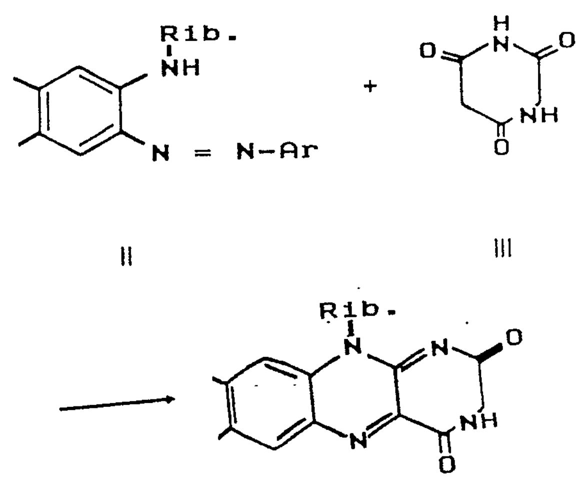<br>II III I |
| ein pH-Wert zwischen 6,5 und 0,8 | 2 | 59 | ein pH-Wert zwischen 6,5 und 0,8 einstellte, |
| 25%-igen wäßrigen NaOH versetzt) | 6 | 32 | 25%-igen wäßrigen NaOH versetzt, |

Tag der Entscheidung über die Berichtigung / Date of decision on rectification: / Date de décision portant sur modification: 02.03.90

Ausgabe- und Veröffentlichungstag: / Issue and publication date: / Date d'edition et de publication: 16.05.90

Patbl.Nr) / EPB no:) / Bull. no:) 90/20

(19) Europäisches Patentamt
European Patent Office
Office européen des brevets

(11) Veröffentlichungsnummer: **0 164 704**
**B1**

(12) # EUROPÄISCHE PATENTSCHRIFT

(45) Veröffentlichungstag der Patentschrift: **06.12.89**

(51) Int. Cl.⁴: **C 07 D 475/14**

(21) Anmeldenummer: **85107062.3**

(22) Anmeldetag: **07.06.85**

(54) **Verfahren zur Reinigung von Riboflavin.**

(30) Priorität: **12.06.84 DE 3421714**

(43) Veröffentlichungstag der Anmeldung: **18.12.85 Patentblatt 85/51**

(45) Bekanntmachung des Hinweises auf die Patenterteilung: **06.12.89 Patentblatt 89/49**

(84) Benannte Vertragsstaaten: **CH DE FR GB LI NL**

(56) Entgegenhaltungen: **US-A-2 822 361**

(73) Patentinhaber: **BASF Aktiengesellschaft, Carl-Bosch- Strasse 38, D-6700 Ludwigshafen (DE)**

(72) Erfinder: **Grimmer, Johannes, Aeblerose Vey 11, DK- 8500 Grenaa (DK)**
Erfinder: **Horn, Hans Christoph, Dr., Roemerstrasse 22, D-6715 Lambsheim (DE)**

**EP 0 164 704 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft ein Verfahren zur Hochreinigung von rohem Riboflavin (I; Vitamin $B_2$) durch Lösen in verdünnter wäßriger Alkalilauge, ggf. Reinigen der alkalischen Lösung und Isolieren des Riboflavins durch Eintragen der Lösung in 90 bis 100°C warme wäßrige Säurelösung.

Riboflavin wird üblicherweise, wie allgemein bekannt ist (vgl. z. B. W. H. Sebrell et R. S. Harris, "The Vitamins; Chemistry, Physiologie, Pathology, Methods", 2. Auflage, Band V, 1982, Academic Press, Seite 22) durch Kondensation eines N-(D)-Ribityl-2-arylazo-4,5-dimethylanilins (II) mit Barbitursäure (III) synthetisch hergestellt.

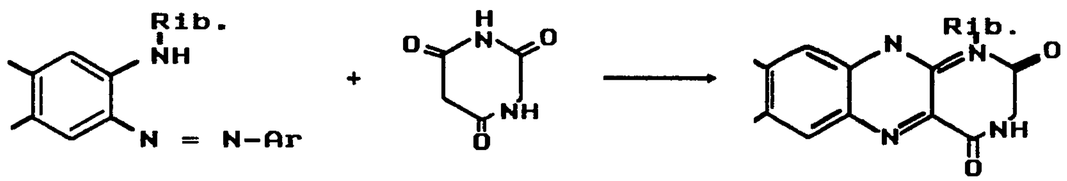

II III I

Rib = D-Ribityl
Ar = Aryl, z. B. Phenyl

Hierbei erhält man ein Rohprodukt, welches neben etwa 92 bis 96 Gew.-% I noch verschiedene Verunreinigungen enthält, darunter beispielsweise Barbitursäure, Dibarbitursäure, Lumiflavin, Lumichrom und N-(D)-Ribityl-6-arylazo-4 ,5-dimethylanilin.

Auch die Kondensation anderer N-(D)-Ribityl-4,5-dimethyl-anilinderivate mit Barbitursäurederivaten zu Riboflavin wurde schon beschrieben.

Literaturbekannt ist überdies die Bildung von Riboflavin auf biosynthetischem Weg mit Hilfe von Mikroorganismen.

Da sich reines Riboflavin durch Kristallisationsvorgänge allein nur mit unverhältnismäßig hohem Aufwand aus der Rohware gewinnen läßt, unterwirft man das rohe Riboflavin nach der Lehre der US-PS-2 324 800 einer oxidativen Behandlung in wäßrig-saurem Milieu, wonach man eventuelle Niederschläge abtrennt und die verbleibende Lösung mit viel Wasser verdünnt. Hierbei fällt das Riboflavin in Form von gelben Nadeln an, die nur noch abfiltriert und gewaschen zu werden brauchen.

Nachteilig an diesem Verfahren ist, daß bei diesem Reinigungsverfahren etwa 15 % des eingesetzten Riboflavins verlorengehen, daß diese Arbeitsweise zeitraubend und technisch umständlich ist und daß das erhaltene I noch immer Spuren von organischen Verbindungen, wie Anilin enthält, die seinen Einsatz für die menschliche Ernährung problematisch machen.

Weiterhin ist aus der US-PS-2 822 361 ein Verfahren zur Gewinnung von Riboflavin aus von Biomasse befreiten Fermentationsausträgen der mikrobiologischen Riboflavinherstellung bekannt, bei dem das Riboflavin bei einem pH-Wert unterhalb 7,5 reduziert wird, das zusammen mit Verunreinigungen augefallene Riboflavin mit wäßriger Alkalihydroxidlösung gelöst wird, die alkalische Lösung mit Sauerstoffgas oxidiert wird, dabei ausfallende Verunreinigungen abfiltriert werden und das Riboflavin durch Ansäuern auf einen pH-Wert unterhalb von 8 ausgefällt wird. Das bei diesem Verfahren erhaltene Riboflavin enthält noch größere Mengen an Verunreinigungen und ist daher für einen Einsatz in der menschlichen Ernährung nicht geeignet.

Es war daher die Aufgabe der Erfindung, ein Verfahren zur Hochreinigung von I zu entwickeln, das die Nachteile des Standes der Technik überwindet, d. h. das technisch vorteilhafter gestaltet werden kann, bei dem weniger I während des Reinigungsprozesses verlorengeht und bei welchem noch reineres I erhalten wird.

Gegenstand der Erfindung ist dementsprechend ein Verfahren zur Reinigung von rohem Riboflavin, das dadurch gekennzeichnet ist, daß man

a) das zu reinigende Riboflavin in Wasser suspendiert und durch Zugabe der wäßrigen Lösung eines Alkalihydroxides in Lösung bringt oder aber das Riboflavin in etwa 0,16 bis 0,63 molarer wäßriger Alkalilauge löst,
b) die erhaltene alkalische Riboflavinlösung ggf. durch Behandeln mit Aktivkohle oder einem Filterhilfsmittel und anschließende Filtration oder durch Extraktion mit einem mit Wasser nicht oder nur wenig löslichen inerten Lösungsmittel reinigt,
c) die erhaltene alkalische Riboflavinlösung unter Aufrechterhalten von Temperaturen zwischen 40 und 100°C, vorzugsweise etwa 97 bis 99°C, in etwa 90 bis 100°C heißes Wasser einträgt, dem soviel einer Säure zugesetzt wurde, daß sich in dem Reaktionsgemisch ein pH-Wert zwischen 6,5 und 0,8
d) das Reaktionsgemisch noch etwa 10 bis 80 Minuten, vorzugsweise 20 bis 60 Minuten, ggf. unter Rühren bei Temperaturen von etwa 90 bis 100°C erhitzt und
e) nach Abkühlen des Reaktionsgemisches das auskristallisierte Riboflavin isoliert.

Besonders vorteilhaft gestaltet sich das Verfahren, wenn die Temperatur der alkalischen Riboflavinlösung in den Reaktionsschritten a) und b) etwa 10 bis 50°C, vorzugsweise etwa 30 bis 45°C, beträgt bzw. die Temperatur von 50°C

höchstens kurzzeitig überschritten wird.

Bei dem erfindungsgemäßen Reinigungsverfahren sind die I-Verluste deutlich geringer als bei einer Reinigung in saurer Lösung. Dies ist sehr überraschend, da in der Literatur über I, selbst in der jüngsten Literatur (vgl. Fermente - Hormone - Vitamine, Band III/1, Georg Thieme Verlag Stuttgart, 1974, Seite 631 und Ullmanns Encyklopädie der technischen Chemie, Band 23, Verlag Chemie, Weinheim, 1983, Seite 664), steht, daß I in alkalischer Lösung leicht zersetzlich ist.

Aus US 2 603 633 ist zwar ein Verfahren zur Herstellung von 3 verschiedenen Typen von I-Kristallen bekannt, gemäß dem die I-Kristalle durch Zugabe von Säuren zu einer alkalischen I-Lösung bei Temperaturen von 10 bis 25°C erhalten werden. Versuche, die beschriebenen Kristallisationsverfahren zur Reinigung von I in technischem Maßstab zu verwenden, ergaben jedoch keine akzeptablen Ergebnisse.

Demgegenüber erhält man gemäß dem erfindungsgemäßen Verfahren Riboflavin in einer Ausbeute von 90 bis 92 % der Theorie und in einer Reinheit von über 99,5 % (bestimmt nach der Pharmakopoe Europa).

Das erfindungsgemäße Verfahren ist geeignet zur Hochreinigung von rohem I mit einem I-Gehalt von etwa 10 bis 99,5 %, wie es bei der mikrobiologischen oder der synthetischen Herstellungsweise anfällt.

Zur Durchführung des erfindungsgemäßen Verfahrens wird das rohe I zunächst in Wasser suspendiert. Man verwendet dabei im allgemeinen eine Wassermenge von etwa 1130 bis 6580 g, vorzugsweise etwa 1880 bis 3760 g pro Mol I. Mit Vorteil verwendet man Wasser von einer Temperatur zwischen 25 und 50°C, vorzugsweise zwischen 35 und 45°C.

Zu dieser wäßrigen I-Suspension wird soviel einer wäßrigen Lösung eines Alkalihydroxides zugefügt, daß das suspendierte I völlig in Lösung geht. Als wäßrige Lösung eines Alkalihydroxides verwendet man vorzugsweise wäßrige KOH oder NaOH, insbesondere die technisch sehr leicht verfügbare und billige NaOH in Form einer etwa 25 %-igen Lösung. Zur Lösung des I benötigt man etwa 1 bis 1,25 Mol Alkalihydroxid pro Mol I, was für 25 %-ige NaOH etwa 0,5 kg pro kg I bedeutet. Man kann aber auch das Riboflavin gleich in verdünnter wäßriger Alkalilauge lösen. Hierzu verwendet man eine etwa 0,16 bis 0,63 molare, vorzugsweise eine etwa 0,28 bis 0,32 molare wäßrige KOH oder NaOH in Mengen von etwa 17,5 bis 5, vorzugsweise 10 bis 9 kg pro kg I, je nach der Konzentration der eingesetzten Alkalilauge.

Das Lösen des I wird im allgemeinen bei Temperaturen zwischen etwa 10 und 50, vorzugsweise 25 und 50° C, insbesondere 30 bis 45° C, vorgenommen. Wird I nur kurzfristig der Einwirkung der wäßrig alkalischen Lösung ausgesetzt, wie dies bei kontinuierlicher Verfahrensführung der Fall ist, können auch Temperaturen höher als 50°C, beispielsweise 50 bis 60°C angewandt werden.

Die erhaltene wäßrig-alkalische I-Lösung kann nun gereinigt werden. Dies kann durch Behandlung mit Aktivkohle und anschließende Filtration oder aber durch Extraktion mit einem mit Wasser nicht oder nur schlecht mischbaren inerten Lösungsmittel erfolgen. Als Aktivkohle können praktisch alle im Handel erhältlichen Sorten eingesetzt werden.

Als Lösungsmittel für die Extraktion kommen beispielsweise Ethylacetat, Chloroform oder Petrolether, insbesondere iso-Butylacetat und n-Butylacetat in Betracht.

Bei der Reinigung von auf mikrobiologischem Wege hergestelltem Roh-I wird eine Reinigung der wäßrig-alkalischen I-Lösung bevorzugt durch Behandeln mit einem Filterhilfsmittel und anschließende Filtration durchgeführt. Die Art des Filterhilfsmittels ist ohne Belang. Wichtig ist nur, daß eine ausreichend große Filtriergeschwindigkeit gewährleistet ist. Bewährt haben sich beispielsweise Celite$^R$ der Firma Johns-Manville, USA.

Die erhaltene, ggf. gereinigte I-Lösung wird anschließend unter Aufrechterhalten von Temperaturen zwischen 90 und 100°C, vorzugsweise zwischen 96 und 99°C, in 96 bis 100°C, vorzugsweise 96 bis 99°C, heißes Wasser eingetragen, den soviel einer Säure zugesetzt wurde, daß sich in dem Reaktionsgemisch ein pH-Wert zwischen 6,5 und 0,8, vorzugsweise zwischen 6 und 1 einstellt. In Sonderfällen, insbesondere bei der Reinigung von auf mikrobiellem Wege hergestelltem Riboflavin, ist es auch möglich, die ggf. gereinigte I-Lösung unter Aufrechterhalten etwas niedrigerer Temperaturen, also zwischen etwa 40 und 100°C, in das angesäuerte heiße Wasser einzutragen.

Die Menge des Wassers wird so bemessen, daß das Reaktionsgemisch nach Zugabe der alkalischen Lösung etwa 18 bis 30 kg Wasser, vorzugsweise etwa 20 kg - 25 kg Wasser pro kg I enthält.

Als Säuren, die dem Wasser zugesetzt werden, eignen sich alle Säuren, die mindestens so stark sind wie Essigsäure, d. h. einen $pK_s$-Wert von 4,76 oder kleiner aufweisen und das Riboflavin unter den Reaktionsbedingungen nicht angreifen. Mit Vorteil verwendet man die üblichen Mineralsäuren, wie HCl ($pK_s$-Wert -6), $H_2SO_4$ ($pK_s$-Wert -3), $HNO_3$ ($pK_s$-Wert -1,32), $H_3PO_4$ ($pK_s$-Wert 2,09). Aber auch organische Säuren, wie Ameisensäure ($pK_s$-Wert 3,77) und Essigsäure ($pK_s$-Wert 4,76) sind verwendbar. Als besonders vorteilhaft erwies sich die Verwendung von, Salpetersäure, da sie offenbar auch in stark verdünnter wäßriger Lösung auf die Nebenprodukte in I einen milden oxidierenden Einfluß hat. Bei Verwendung von $HNO_3$ erhält man reines I in einer Farbe, die sich von der unter Verwendung der anderen Säuren erzielten Farbe in ihrer Brillianz deutlich unterscheidet.

Die Mineralsäure wird in solchen Mengen verwendet, daß einerseits die eingesetzte Alkalilauge völlig neutralisiert und andererseits die Lösung sauer reagiert, daß das I vorteilhaft und vollständig ausfällt. Bei Reinigungsansätzen ohne zwischengeschaltete Behandlung mit Aktivkohle oder Extraktionsmittel empfiehlt es sich, den Ansatz bis zu einem pH-Wert von unter 3 anzusäuern, um eventuelle Nebenprodukte während des Erhitzens leichter zu zerstören.

Im allgemeinen sind dazu je nach der verwendeten Menge Alkalilauge etwa 1,06 bis 3,5 Mol Säure pro Mol I notwendig.

Die effektive I-Ausbeute bei dem Reinigungsverfahren kann noch dadurch verbessert werden, daß man zu

der erfindungsgemäß erhaltenen wäßrigsauren Lösung im Reaktionsschritt e) noch für den jeweils vorgesehenen Verwendungszweck in der Tierernährung bzw. Pharmaindustrie zugelassene Ballaststoffe zusetzt.

Den vorteilhaften Effekt kann man wohl so erklären, daß hierbei das Adsorptionsverhalten von I so ausgenutzt wird, daß man die an dem Roh-I adsorbierten 4 bis 7 % Nebenprodukte durch unlösliche, in der Tierernährung bzw. Pharmaindustrie zugelassene Zusatzstoffe ersetzt.

Als von der Pharmaindustrie zugelassene Ballaststoffe seien beispielsweise Maisstärke, Maisspindelmehl, Sojafeinmehl und Weizengrießkleie genannt. In der Tierernährung zugelassene Zusatzstoffe sind beispielsweise $SiO_2$, Calciumsilicate, Kieselgur, Steatit, Talkum und Bolus alba (weißer Ton). Die Zusatzstoffe verwendet man im allgemeinen in Mengen von 1 bis 500 g pro kg I.

**Beispiel 1**

Abhängigkeit der I-Ausbeute von der Alkalilaugekonzentration.

Jeweils 40 g Riboflavin wurden in 400 ml Wasser suspendiert, die Suspension auf 40°C erwärmt, in die Suspension die aus der Tabelle 1 ersichtliche Menge einer 25 %-igen NaOH eingetragen und das Reaktionsgemisch 15 Minuten (Min.) bei 40°C gerührt. Anschließend wurde die alkalische Lösung innerhalb von 20 Min. in eine 98°C heiße Mischung aus 400 ml Wasser und der aus der Tabelle 1 ersichtlichen Menge einer konzentrierten (37 %-igen) Salzsäure eingetragen, die alkalischen Rückstände mit Wasser nachgespült, das Reaktionsgemisch 1 Stunde (h) bei 98 - 100°C gerührt, dann auf 40°C abgekühlt, der Niederschlag abgesaugt und mit 400 ml eines 60°C warmen Wassers und 200 ml Methanol gewaschen. Die Trocknung erfolgte bei 80 - 100°C. Aus der durch Auswaage bestimmten I-Menge wurde die Ausbeute berechnet.

**Tabelle 1**

| Beispiel | 25 %-ige NaOH [g] | 37 %-ige HCl [g] | Ausbeute [g] | Ausbeute [% der Theorie] |
|---|---|---|---|---|
| 1 a | 20 | 30 | 38,7 | 96,75 |
| 1 b | 40 | 60 | 38,4 | 96,00 |
| 1 c | 60 | 90 | 37,8 | 94,50 |

**Beispiel 2**

I-Ausbeute in Abhängigkeit von der Temperatur der alkalischen I-Lösung.

Jeweils 40 g I wurden in 400 ml Wasser suspendiert, die Suspension mit 20 g einer 25 %-igen NaOH-Lösung versetzt und dann 15 Min. unter Rühren auf die aus Tabelle 2 ersichtliche Temperatur erhitzt. Die Aufarbeitung des Reaktionsansatzes sowie die Ausbeutebestimmung erfolgte analog Beispiel 1 a.

**Tabelle 2**

| Beispiel | Temperatur [°C] | Ausbeute [g] | Ausbeute [% der Theorie] |
|---|---|---|---|
| 2 a | 30 | 38,7 | 96,75 |
| 2 b | 40 | 38,8 | 97,00 |
| 2 c | 50 | 38,4 | 96,00 |
| 2 d | 60 | 37,7 | 94,25 |
| 2 e | 70 | 34,9 | 87,25 |

**Beispiel 3**

I-Ausbeute in Abhängigkeit von der HCl-Konzentration.

Jeweils 40 g I wurden in 400 ml Wasser suspendiert, die Suspension auf 40°C erwärmt, 20 g einer 25 %-igen NaOH-Lösung eingetragen und das Reaktionsgemisch 15 Min. unter Rühren auf 40°C erwärmt. Anschließend wurde die alkalische Lösung innerhalb von 20 Min. in eine 98°C heiße Mischung aus 400 ml Wasser und der aus Tabelle 3 ersichtlichen Menge einer 37 %-igen HCl eingetragen. Die Aufarbeitung des Reaktionsansatzes sowie die Ausbeutebestimmung erfolgten analog Beispiel 1 a

**Tabelle 3**

| Beispiel | 37%-ige HCl [g] | Ausbeute [g] | [% der Theorie] |
|---|---|---|---|
| 3 a | 30 | 38,9 | 97,25 |
| 3 b | 40 | 38,6 | 96,50 |
| 3 c | 50 | 38,3 | 95,75 |
| 3 d | 60 | 38,5 | 96,25 |

**Beispiel 4**

Zusätzliche Reinigung mit Aktivkohle.

40 g eines Rohriboflavins (93 %-ig) wurden in 400 ml Wasser bei 40°C suspendiert, die Suspension mit 20 g einer 25 %-igen NaOH und danach mit 1 g Aktivkohle vom Typ "2 S" der Firma Chemviron versetzt und unter Rühren 15 Min. auf 40°C erwärmt. Anschließend wurde die Lösung über eine G4 Glasfilternutsche abgesaugt und der Rückstand mit Wasser gewaschen. Die vereinigten Filtrate wurden innerhalb von 30 Min. in eine 98°C heiße Mischung aus 400 ml Wasser und 30 g einer 37 %-igen HCl gepumpt, das Reaktionsgemisch noch 1 h unter Rühren auf 98 - 100°C erhitzt, dann auf 40°C abkühlen lassen und I analog Beispiel 1 a isoliert.

Die Ausbeute betrug 35,7 g, entsprechend 89,25 % der Theorie, die Reinheit 99,9 % (Pharm. Europ.), der Anilingehalt etwa 3 ppm.

**Beispiel 5**

Zusätzliche Extraktion mit iso-Butylacetat.

Jeweils 40 g Roh-I (93 %-ig) wurden in 500 ml Wasser suspendiert, die Suspension mit 20 g einer 25 %-igen NaOH-Lösung versetzt und die erhaltene Lösung noch 15 Min. bei Raumtemperatur (RT) gerührt. Anschließend wurde mit 150 ml iso-Butylacetat und dann mit 100 ml iso-Butylacetat ausgeschüttelt. Nach Abtrennen der organischen Phasen wurde die wäßrig alkalische Lösung in eine etwa 98°C heiße Mischung aus 300 ml Wasser und 30 g einer 37 %-igen HCl eingetragen, das Reaktionsgemisch noch 1 h unter Rühren auf 98 - 100°C erhitzt, dann abkühlen lassen und analog Beispiel 1 aufgearbeitet.

Die Ausbeute betrug 35,3 g entsprechend 88,25 % der Theorie, die Reinheit 100,1 % (Pharm. Europ.).

Die abgetrennte organische Phase enthielt 1,3 g Trockensubstanz, die etwa zur Hälfte aus dem für die I-Herstellung unbrauchbaren N-(D)-Ribityl-6-phenylazo-4,5-dimethylanilin sowie einer Vielzahl nicht näher untersuchter Produkte bestand.

**Beispiel 6**

a) Alkalische Reinigung unter Zusatz von Maisstärke zu I.

40 g Roh-I (93 %-ig) wurden in 400 ml Wasser suspendiert, die Suspension auf 40°C erwärmt, mit 20 g einer 25 %-igen NaOH und danach mit 1 g Aktivkohle versetzt und das erhaltene Gemisch unter Rühren 15 Min. auf 40°C gehalten. Anschließend wurde die Kohle über ein G - 4 Glasfilter abgetrennt, der Rückstand mit Wasser riboflavinfrei gewaschen, die vereinigten alkalischen Filtrate innerhalb von 8 Min. in eine 98°C heiße Mischung aus 400 ml Wasser und 30 g konz. HCl eingetragen, das Reaktionsgemisch 1 h unter Rühren bei 98°C gehalten, dann auf 50°C abgekühlt und mit 1,5 g Maisstärke versetzt. Nach 15 Min. Rühren bei 40°C wurde abgesaugt, das Produkt analog Beispiel 1 a mit Wasser und Methanol gewaschen und bei 90° - 100°C getrocknet.

Die Ausbeute betrug 36,8 g entsprechend einer Nettoausbeute von 98,9 % (unter Berücksichtigung der Maisstärke).

b) Salzsaure Reinigung unter Zusatz von Maisstärke zu I.

40 g Roh-I (93 %-ig) wurden langsam in 66 g konz. HCl eingetragen, nach etwa 3 Min. mit 27,5 g Wasser versetzt und die erhaltene Lösung 30 Min. unter Rühren auf 32 bis 34°C gehalten. Anschließend wurde die salzsaure I-Lösung innerhalb von 9 Min. in 800 ml etwa 98°C heißen Wassers eingetragen und das Reaktionsgemisch für 1 h auf 98°C gehalten. Nach Abkühlen auf 50°C wurde mit 1,5 g Maisstärke versetzt, 15 Min. unter Rühren auf 40°C gehalten und dann das I abgesaugt, analog Beispiel 1 a gewaschen und getrocknet.

Ausbeute: 36,0 g, entsprechend einer Nettoausbeute von 96,8 %.

**Beispiele 7 - 11**

Jeweils die aus Tabelle 4 ersichtliche Menge an feuchtem Roh-I (entsprechend immer 40 g an trockenem Roh-I; 93,8 %-ig) wurde in 400 ml Wasser suspendiert, die Suspension auf die aus der Tabelle ersichtliche Temperatur erwärmt und mit 20 g einer 25 %-igen NaOH versetzt. Nach 15 Min. Rühren bei der angegebenen Temperatur wurde die alkalische Lösung innerhalb von 30 Min. in eine 98°C heiße Mischung aus 400 ml Wasser und der aus Tabelle 4 ersichtlichen Mineralsäure eingetragen, wobei sich der aus der Tabelle 4 ersichtliche pH-Wert im Reaktionsgemisch einstellte. Anschließend wurde das Reaktionsgemisch 1 Stunde unter Rühren bei 98 - 100°C gehalten, danach auf 40°C abgekühlt, der Niederschlag abgesaugt, mit 400 ml eines 60°C warmen Wassers und 200 ml Methanol gewaschen und bei 80 - 100°C getrocknet. Die I-Ausbeuten und I-Reinheiten sind in der Tabelle 4 angegeben. Die Reinheiten wurden nach der Vorschrift des Europäischen Arzneibuchs, Bd. 1 (Pharm. Europ.) bestimmt.

**Tabelle 4**

| Beispiel | feuchtes Roh-I [g] | Säure | pH-Wert | Temperatur [°C] | Ausbeute [g] | Ausbeute (% d.Theorie) | Reinheit [g] |
|---|---|---|---|---|---|---|---|
| 7 | 48,7 | 34,8 g $HNO_3$ 65 %-ig | 0,85 | 30 | 35,8 | 95,4 | 100,5 |
| 8 | 49,1 | 31,0 g $H_2SO_4$ 96 %-ig | 0,84 | 40 | 35,1 | 93,55 | 100,5 |
| 9 | 49,1 | 35,1 $H_3PO_4$ g 85 %-ig | 0,98 | 40 | 35,8 | 95,4 | >99,9 |
| 10 | 49,0 | 14 g Ameisensäure 100 %-ig | 3,5 | 40 | 34,5 | 92,1 | >99,9 |
| 11 | 49,0 | 18,3 g Eisessig | 4,5 | 40 | 34,0 | 90,6 | >99,9 |

**Beispiel 12**

Reinigung von fermentativ hergestelltem Riboflavin.

50 g eines auf fermentativem Wege hergestellten Riboflavins (62 %-ig) und 20 g des Filterhilfsmittels Celite (Standard Super-Cel$^R$ der Firma Johns-Manville, USA) wurden in 500 ml Wasser bei 20°C suspendiert, die Suspension mit 18 g einer 25 %-igen wäßrigen NaOH versetzt) 15 Min. gerührt und anschließend filtriert. Der Filterkuchen wurde mit 200 ml Wasser riboflavinfrei gewaschen. Das Waschwasser wurde mit der Mutterlauge vereinigt und das Ganze innerhalb von 30 Min. in eine 98 bis 100°C heiße Mischung aus 100 ml Wasser und 27 g konzentrierter Salzsäure gepumpt. Nach einstündigem Rühren bei 98 - 100°C wurde langsam auf 40°C abgekühlt, das abgeschiedene Riboflavin abgesaugt, neutral gewaschen und getrocknet.

Die Ausbeute betrug 30 g, entsprechend 96,7 % der Theorie, bezogen auf die Rohware, die Reinheit 100,1 % (Pharm. Europ.).

**Patentansprüche**

1. Verfahren zur Reinigung von rohem Riboflavin, dadurch gekennzeichnet, daß man

   a) das zu reinigende Riboflavin in Wasser suspendiert und durch Zugabe der wäßrigen Lösung eines Alkalihydroxides in Lösung bringt oder aber das Riboflavin in etwa 0,16 bis 0,63 molarer wäßriger Alkalilauge löst,
   b) die erhaltene alkalische Riboflavinlösung ggf. durch Behandeln mit Aktivkohle oder einem Filterhilfsmittel und anschließende Filtration oder durch Extraktion mit einem mit Wasser nicht oder nur wenig löslichen inerten Lösungsmittel reinigt,
   c) die erhaltene alkalische Riboflavinlösung unter Aufrechterhalten von Temperaturen zwischen 40 und 100°C in 90 bis 100°C heißes Wasser einträgt, dem soviel einer Säure zugesetzt wurde, daß sich in dem Reaktionsgemisch ein pH-Wert zwischen 6,5 und 0,8 einstellt,
   d) das Reaktionsgemisch noch etwa 10 bis 80 Minuten ggf. unter Rühren auf Temperaturen von etwa 90 bis 100°C erhitzt und
   e) nach Abkühlen des Reaktionsgemisches das auskristallisierte Riboflavin isoliert.

2. Verfahren gemäß Anspruch 1, dadurch gekennzeichnet, daß man die Reaktionsschritte a) und b) bei Temperaturen zwischen 30 und 45°C durchführt.

3. Verfahren gemäß Anspruch 1, dadurch gekennzeichnet, daß man im Reaktionsschritt e) dem Reaktionsgemisch noch für den jeweils vorgesehenen Verwendungszweck in der Tierernährung bzw. Pharmaindustrie zugelassene Zusatzstoffe zusetzt.

4. Verfahren gemäß Anspruch 1, dadurch gekennzeichnet, daß man die alkalische Riboflavinlösung im Reaktionsschritt c) unter Aufrechterhalten von Temperaturen zwischen 90 und 100°C in das heiße Wasser einträgt.

5. Verfahren gemäß Anspruch 1, dadurch gekennzeichnet, daß man die im Reaktionsschritt a) erhaltene alkalische Riboflavinlösung durch Behandeln mit einem Filterhilfsmittel und anschließende Filtration reinigt.

**Claims**

1. A process for the purification of crude riboflavin, wherein

a) the riboflavin to be purified is suspended in water and brought into solution by the addition of an aqueous solution of an alkali metal hydroxide, or the riboflavin is dissolved in about 0.16 - 0.63 molar aqueous alkali metal hydroxide solution,
b) if necessary, the resulting alkaline riboflavin solution is purified by treatment with active carbon or a filtration aid followed by filtration, or by extraction with an inert solvent which is insoluble or only slightly soluble in water,
c) while a temperature of from 40 to 100°C is maintained, the alkaline riboflavin solution obtained is introduced into water which is at 90 - 100°C and to which an acid has been added in an amount such that the pH of the reaction mixture is brought to 6.5 - 0.8,
d) the reaction mixture is heated at about 90 - 100°C for about a further 10 - 80 minutes with or without stirring, and
e) the reaction mixture is cooled and the riboflavin which has crystallized out is isolated.

2. A process as claimed in claim 1, wherein the reaction steps a) and b) are carried out at from 30 to 45°C.

3. A process as claimed in claim 1, wherein, in reaction step e), additives which are permitted for the particular intended use in animal nutrition or the pharmaceutical industry are also added to the reaction mixture.

4. A process as claimed in claim 1, wherein, in reaction step c), the alkaline riboflavin solution is introduced into the hot water while maintaining a temperature of from 90 to 100°C.

5. A process as claimed in claim 1, wherein the alkaline riboflavin solution obtained in reaction step a) is purified by treatment with a filtration aid followed by filtration.

**Revendications**

1. Procédé de purification de riboflavine brute caractérisé par le fait que:

a) on suspend dans de l'eau la riboflavine à purifier et on la met en solution par addition de la solution aqueuse d'un hydroxyde alcalin, ou bien on dissout la riboflavine dans une lessive alcaline aqueuse environ 0,16 à 0,63 molaire;
b) on purifie la solution de riboflavine alcaline obtenue, éventuellement par traitement avec du charbon actif ou un auxiliaire de filtration et ensuite filtration, ou par extraction avec un solvant inerte non ou peu soluble dans l'eau;
c) on introduit la solution de riboflavine alcaline obtenue dans de l'eau chaude à 90 à 100°C, en maintenant des températures comprises entre 40 et 100°C, à laquelle a été ajoutée une quantité d'acide telle qu'il s'établit dans le mélange de réaction un pH compris entre 6,5 et 0,8;
d) on chauffe le mélange de réaction encore 10 à 80 minutes, éventuellement en agitant, à des températures d'environ 90 à 100°C et,
e) après refroidissement du mélange de réaction, on isole la riboflavine qui a cristallisé.

2. Procédé selon la revendication 1, caractérisé par le fait que l'on exécute les étapes de réaction a) et b) à des températures comprises entre 30 et 45°C.

3. Procédé selon la revendication 1, caractérisé par le fait que, dans l'étape de réaction e), on ajoute encore au mélange de réaction des additifs admissibles pour les différents buts d'utilisation prévus dans l'alimentation des animaux ou l'industrie pharmaceutique.

4. Procédé selon la revendication 1, caractérisé par le fait que l'on introduit la solution de riboflavine alcaline, dans l'étape de réaction c), dans de l'eau chaude en maintenant des températures comprises entre 90 et 100°C.

5. Procédé selon la revendication 1, caractérisé par le fait que l'on purifie la solution de riboflavine alcaline, obtenue dans l'étape de réaction a), par traitement avec un auxiliaire de filtration et ensuite filtration.